# EUROPEAN PATENT SPECIFICATION

(11) **EP 2 604 273 B1**
(45) Date of publication and mention of the grant of the patent: **08.10.2014**
(21) Application number: 11009852.2
(22) Date of filing: 14.12.2011
(51) Int. Cl.: A61K 36/185, A61K 36/53, A61P 31/04

(54) **Composition for the treatment of bacterial infections**
Zusammensetzung zur Behandlung von Bakterieninfektionen
Composition pour le traitement des infections bactériennes

(43) Date of publication of application: 19.06.2013
(73) Proprietor: Pandalis, Georgios, 49219 Glandorf (DE)
(72) Inventor: Pandalis, Georgios, 49219 Glandorf (DE)
(74) Representative: Grünecker, Kinkeldey, Stockmair & Schwanhäusser

(56) References cited:
- DE-U1-202004 015 396
- anonymous: "Immupower", , 6 October 2010 (2010-10-06), XP002677761, Retrieved from the Internet: URL:http://www.heavenscentoils.net/immupow er.htm [retrieved on 2012-06-14]
- Anonymous: "Young Living ImmuPower Essential Oil Blend", , 1 October 2011 (2011-10-01), XP002677762, Retrieved from the Internet: URL:http://aliciawarwick.wordpress.com/201 1/10/21/10-off-immupower-oil-blend-get-rid -of-flu-infections/ [retrieved on 2012-06-14]
- KINTZIOU H ET AL: "ANTIMICROBIAL ACTIVITY OF ESSENTIAL OILS FROM GREEK PLANTS: APPLICATIONS AND THERAPEUTIC USES", EPITHEORESE KLINIKES FARMAKOLOGIAS KAI FARMAKOKINETIKES - REVIEWOF CLINICAL PHARMACOLOGY AND PHARMACOKINETICS, ATHENS, GR, vol. 15, no. 1, 1 January 2001 (2001-01-01), pages 15-20, XP009017039, ISSN: 1011-6583
- CHINOU I ET AL: "CYTOTOXIC AND ANTIBACTERIAL LABDANE-TYPE DITERPENES FROM THE AERIALPARTS OF CISTUS INCANUS SUBSP. CRETICUS", PLANTA MEDICA, THIEME VERLAG, DE, vol. 60, no. 1, 1 January 1994 (1994-01-01), pages 34-36, XP000645961, ISSN: 0032-0943
- CAVANAGH HEATHER M A ET AL: "Antibacterial activity of berry fruits used for culinary purposes.", JOURNAL OF MEDICINAL FOOD SPRING 2003 LNKD- PUBMED:12804021, vol. 6, no. 1, April 2003 (2003-04), pages 57-61, XP002677763, ISSN: 1096-620X

## Description

The present invention relates to a composition comprising a concentrate or an extract of origanum for the treatment of bacterial infections in combination with at least one concentrate or extract of plants of the family Grossulariaceae and Cistaceae.

A great number of diseases are caused by bacterial infection. The fight against bacterial infection represents one of the high points in modern medicine. The development of antibiotics in the 1940s offered physicians a powerful tool against bacterial infections that has saved the lives of millions of people. However, because of the widespread and sometimes inappropriate use of antibiotics, strains of bacteria that are antibiotica-resistant have begun to emerge. These new, stronger bacteria pose a significant threat to general welfare and health.

Bacterial infections can be caused by a wide range of bacteria, resulting in mild to life-threatening illnesses (such as bacterial meningitis) that require immediate intervention. Common bacterial infections include for example pneumonia, ear infections, diarrhea, urinary tract infections, and skin disorders.

The treatment of bacterial infections is most often achieved by using antibiotics which either aim at killing invading bacteria (bactericide mode of action) or inhibiting their growth (bacteriostatic mode of action) without harming the host. Antibiotic effectiveness depends on mechanism of action, drug distribution, site of infection, immune status of the host, and resistance factors of bacteria. Antibiotics work through several mechanisms; some inhibit the formation of bacterial cell walls. Others interrupt bacterial protein synthesis. Yet some others inhibit metabolism or interfere with DNA synthesis and/or cell membrane permeability.

Since their discovery in the 1940s, many antibiotics have lost their effectiveness against common bacterial infections since bacteria developed resistances against the drugs leading to increased hospitalizations, health costs, and mortality.

In particular, *Staphylococcus aureus,* a facultative anaerobic Gram-positive coccal bacterium, is considered as one of the most dangerous microorganisms of our days by virtue of its resistance against to all common antibiotics including for example vancomycin. *S. aureus* can cause a range of diseases ranging from minor skin infections, such as pimples, impetigo, boils, cellulitis folliculitis, carbuncles, skaled skin syndrome, and abscesses, to life-threatening diseases such as pneumonia, meningitis, osteomyelitis, endocarditis, toxic shock syndrome, bacteraemia, and sepsis. Its incidence is from skin, soft tissue, respiratory, bone, joint, endovascular to wound infections. In particular, methicillin-resistant *S*. *aureus* (MRSA) is troublesome since these organisms have developed resistances not only to β-lactams but also to many other antimicrobial agents. MRSA is most often found in hospitals and nursing homes, where patients with open wounds, invasive devices, and weakened immune systems are at greater risk of infection than the general public.

An alternative approach for the treatment of bacterial infections consists in the use of bacteriostatic nutrients. Origanum, for example, has been known for many centuries for its antibacterial activity.

Origanum essential oil has been used for a long time in far Eastern and Middle Eastern cultures to treat respiratory infections, chronic inflammation, urinary tract infections, dysentery, and jaundice. A large number of in vitro studies have shown origanum oil, or its most active constituents carvacrol and thymol, to inhibit the growth of a broad range of bacteria.

Hammer and colleagues for example investigated 52 plant oils for activity against nine bacteria. Origanum oil was one of only three oils that inhibited the growth of *Pseudomonas aeruginosa,* a hard-to-kill bacterium that causes human wound infections. Overall, origanum oil was better at inhibiting germ growth than all oils tested except lemon grass oil (Hammer, K. et al. J. Appl. Microbial 1999, 86, 985-990).

Baratta and coworkers tested origanum oil besides sage, rosemary, laurel and coriander oils against 25 bacteria. They noted that origanum oil manifested the broadest and highest activity against almost all of the bacteria tested; in fact it strongly inhibited 19 of the 25 bacterial strains under investigation (Baratta, M.T. et al. J. Essent. Oil Res. 1998, 10, 618-627).

In a variety of studies, it has been demonstrated that origanum oil provides efficient activity in the inhibition of growth of *S*. *aureus.*

Celik and co-workers for example investigated the antimicrobial activity of the essential oil of *Origanum hypericifolium* against a number of *Staphylococcus aureus* strains including MRSA strains. Results showed that *O*. *hypericifolium* has the potential for being used in food and medicine because of its antibacterial and antioxidant activity (Celik, A.; Nur Herken, E.; Arslan, I.; Zafer Ozel, M.; Mercan, N. Nat. Prod. Res. 2010, 24, 1568-1577).

Another study investigated the antibacterial activity of the essential oil in comparison to infusion, and decocation derived from Origanum vulgare against 111 gram-positive bacterial isolates belonging to 23 different species related to 3 genera. While essential oil and infusion exhibited antibacterial activity against *S*. *saprophyticus* and *S*. *aureus* inter alia, the isolates were found to be resistant to decoction of origanum (Saeed, S.; Tariq, P; Pak. J. Pharm. Sci. 2009, 22, 421-424).

The antibacterial effects of origanum oils were also demonstrated in *in vivo* studies carried out with mice. Origanum oil was shown to be bactericidal in culture to two strains of *Staphylococcus aureus* (ATCC #14154 and #14775) at 0.25 mg/mL. In vitro, monolaurin's effects mirrored origanum oil. The combination of both was bactericidal at the 0.125 mg/mL concentration of each. In two separate *in vivo* experiments, injected *S. aureus* (ATCC #14775) killed all 14 untreated mice within a 1-week period. In treated mice, over one third survived for 30 days when given oral origanum oil daily for 30 days (6/14). Over 60% of mice survived when receiving a daily combination of origanum oil and monolaurin (5/8). This study shows that origanum oil in combination with monolaurin may prove to be a useful antimicrobial agent for prevention and therapy of S. *aureus* infections (Preuss H.G. et al., Toxicol. Med. Methods 2005, 15(4), 279-285).

Most of the studies investigating the antibacterial properties of origanum use the essential oil obtained by distillation. An extract obtained by dissolving origanum leaves in ethanol has been described by Yoshino and coworkers, who investigated the antioxidant and antiflammatory activities of the origanum extract. However, this study does not relate to the antibacterial properties of origanum (Yoshino K.; Higashi, N., Koga, K. J. of Health Sci. 2006, 52, 169-173).

Further investigations focus on the essential oil of origanum obtained by solvent-free microwave extraction (SFME), supercritical fluid extraction or hydrodistillation. A study conducted by Karakaya et al. revealed that essential oils obtained by solvent-free microwave extraction at different microwave powers and hydrodistillation inhibited the survival of *Listeria monocytogenases, Salmonella typhimurium,* and *Escherichia coli 0157:H7,* whereas survival of *Staphylococcus aureus* was not influenced (Karakaya S. et al., J. Med. Food 2011, 14(6), 645-652).

The above described studies illustrate the dependency of the antimicrobial activity of origanum on the procedure the active compounds are isolated from the plant. According to most of the studies performed, essential origanum oil obtained by distillation seems to provide the greatest potential for inhibiting bacterial growth.

A different strategy to enhance the efficacy of the activity profile of a medical plant consists in the combination of different herbs so as to enhance the efficacy synergistically. For example, WO-A-2010091415 describes an antimicrobial composition containing low concentrations of essential oils and a botanical extract in combination with a fruit acid and alkanediol, and optionally a solvent. This invention, however, is silent about the use of an origanum extract as an optional ingredient and moreover, the composition according to this invention may be used in personal care products such as creams or soap products and not as a drug. US-A-20100092581 relates to an antibacterial composition comprising an extract from a plant belonging to the genus Arceuthobium, such as Arceuthobium Americanum. The composition shows activity against MRSA and may be used in a pharmaceutical composition or a disinfectant. Nonetheless, origanum is not listed in this document as potential ingredient.

A composition consisting of the extracts of horseradish and nasturtium is described in the Arzte Zeitung (16.12.2002). The bacteriostatic activity of said composition against MRSA is derived from the ingredient mustard oil; the composition, however, does not relate at all to origanum.

WO-A-2010049542 is directed to a hydrolyzate of at least one extract of at least one plant material, which shows antibacterial activity, inter alia against MRSA. Although a variety of potential plant materials listed in the document, extracts derived from origanum are not listed.

HeavenScentOils.net, http://www.heavenscentoils.net/immupower.htm, describes an oil blend called "ImmuPower" for building strengthening and protecting the body and supporting its defense mechanism.

Kintziou H. et al., Epitheorese Klinikes Farmakologias Kai Farmakokinetikes, International Edition 2001, 15, 15-20 describe essential oils from Greek plants, their applications and therapeutic uses.

DE-U-20 2004 015 396 discloses a mixture of oregano essential oil and lanolin for eliminating MRSA.

Chinou, I. et al., Planta Med. 1994, 60, 34-36 describe antibacterial labdane-type diterpenes from *Cistus incanus.*

Cavanagh, H.M.A. et al., Journal of Medicinal Food 2003, 6(1), 57-61 describe the antibacterial activity of berry fruits, e.g. raspberry, blackcurrant, cranberry, and blackberry.

It is therefore the object of the present invention to provide a new and effective antibacterial composition for the treatment of bacterial infections. This object is attained by a composition for use in treating bacterial infections, comprising a concentrate or an extract of origanum in combination with at least one concentrate or extract of another plant, wherein the plant is selected from plants of the family *Grossulariaceae* and *Cistaceae:*

The concentrate or extract of origanum according to the invention can be derived from the species *Origanum acutidens, Origanum amanum, Origanum calcaratum, Origanum compactum, Origanum dictamnus, Origanum laevigatum, Origanum leptocladum, Origanum libanoticum, Origanum majorana, Origanum microphyllum, Origanum rotundifolium, Origanum scabrum, Origanum sipyleum, Origanum syriacum, Origanum vulgare.* The preferred origanum species according to the invention is *Origanum vulgare.*

In a preferred embodiment of the invention, in combination with any one of the embodiments listed above or below, the composition in accordance with the present invention is an extract of origanum in combination with at least one extract of another plant.

In a further preferred embodiment of the invention, in combination with any one of the embodiments listed above or below, the concentrate or extract of said composition for use is from the aboveground parts of the plant.

The term "plant" refers to all parts of the plant which are aboveground, including leaves, twigs, blossoms, fruits and seeds.

In another preferred embodiment of the invention, in combination with any one of the embodiments listed above or below, the plant of the composition for use is selected from the genus *Ribes,* and *Cistus.*

In a more preferred embodiment of the invention, in combination with any one of the embodiments listed above or below, the plant of the composition for use is selected from *Ribes nigrum, Ribes rubrum,* and *Cistus incanus.*

For preparing the concentrate or extract according to the present invention, all elements of the aboveground parts of the plant can be used. Preferably, the leaves, twigs and blossoms are used. Preferably, the plant material is coarsely cut.

According to the invention, the term "concentrate" is used representatively for all products that are obtained from a herbal subject by means of removal of water from the fresh plant.

According to the invention, the term "extract" is used representatively for all products that are obtained from a herbal subject by means of an extraction with a solvent, such as maceration or percolation.

As for the extraction, the aboveground parts of the plant are submitted either in the raw state or dried to maceration or percolation. In a preferred embodiment, the dried plant material is used.

The plant parts can be broken into small pieces in a suitable manner before the extraction, by means of rubbing or cutting them, for example. Alternatively, the plant parts can be pressed out directly after the harvest, meaning in the raw state, in order to produce a juice from pressing before the extraction.

Generally, an extraction of the plant parts including leaves, twigs and blossoms is performed with a suitable solvent. Suitable solvents are water, alcohols, such as methanol, ethanol or isopropyl alcohol, or chlorinated solvents, such as dichloromethane, as well as acetone, acetylacetone, ethylacetate, ammonia or glacial acetic acid, but also supercritical carbon dioxide. Mixtures of the solvents mentioned can also be used.

Furthermore, fats, such as pork fat, waxes, such as beeswax, or oils, such as olive oils and almond oil, can be used for the extraction. Preferably, almond oil is used.

In a preferred embodiment of the invention, in combination with any one of the embodiments listed above or below, the extract of the composition for use is an aqueous extract or an alcoholic extract. In a more preferred embodiment, in combination with any one of the embodiments listed below or above, water or a mixture of water with methanol or ethanol is used.

The extraction is normally carried out at temperatures between 25 and 100 °C, depending on the boiling point of the solvent used. Preferred is an extraction at 95 to 100 °C.

In another preferred embodiment of the invention, in combination with any one of the embodiments listed above or below, the extraction is normally carried out for 1 min to 8 h, more preferably 1 to 3 h, in particular the extraction is carried out for 1 h.

In order to achieve the highest possible yield, the plant material can be extracted a number of times. Preferably, the extraction is repeated 2 to 6 times, more preferably 3 times. In this case, it is also possible to use different solvents in the various extraction steps or an extraction with a solvent can be followed by an extraction with fat, wax or oil, or vice versa.

A maceration procedure is normally performed in 5 to 9 days, preferably for 7 days, at room temperature with a mixture of water and ethanol, by pouring the solvent mixture over the plant elements and letting this stand for the period of time mentioned.

According to the invention, a percolation of the plant parts is normally achieved by treating the parts with water at 95 to 100 °C for 4 to 5 hours by conducting the water through the plant parts.

The crude extraction product can also be concentrated and/or dried and/or further processed before use. To produce a dry extract, the solvent can be withdrawn from the liquid raw extract, the concentrated extract or the cleaned extract by, for example, spray drying, freeze drying or vacuum drying. The further processing can, for example, include cleaning steps known to the person skilled in the art, such as centrifugation, filtration and decantation, in order to remove suspended materials from the extract. Chromatography, such as column chromatography, gas chromatography or HPLC or steam distillation also is used for purification. In a preferred embodiment the crude product is used without further purification steps.

The composition for use according to the invention can be produced either by mixing the aboveground parts of *Origanum* and another plant before carrying out the extraction; alternatively and preferably, the concentrate or extract of *Origanum* is mixed with the extract of another plant so as to obtain the composition for use according to the invention.

In a preferred embodiment of the invention, in combination with any one of the embodiments listed above or below, the composition according to the invention comprises a concentrate or an extract of *Origanum* and another plant in a ratio of 1 : 10 to 10 : 1, preferably 1 : 5 to 5 : 1, and more preferably 1 : 2 to 2 : 1 and in particular 1:1.

In a preferred embodiment in combination with any of the above or below embodiments, the composition according to the invention moreover comprises combinations of *Origanum* and two or more other plants. Preferred embodiments are *Origanum*/*Cistus*/*Ribes*, in particular *Origanum vulgare*/*Cistus incanus*/*Ribes nigrum.*

In a preferred embodiment of the invention, in combination with any one of the embodiments listed above or below, the composition according to the invention is in liquid, dry or semi-solid form.

In another preferred embodiment of the invention, in combination with any one of the embodiments listed above or below, the bacterial infection is caused by pathogenous bacteria.

The term "pathogenous bacteria" as used herein refers to bacteria that cause bacterial infections. Pathogenous bacteria include both Gram-positive and Gram-negative bacteria, forming small coccobacilli; small, round, ovoid rods; large, blunt-ended rods; small, slender, pleomorphic rods; ovoid to spherical rods; long, slender, flexible, spiral- or corkscrew-shaped rods; slender, short rods; curved rods with single polar flagellum, and Kidney bean-shaped rods.

In a preferred embodiment of the invention, in combination with any one of the embodiments listed above or below, the bacterial infection is an infection of the gastrointestinal tract, an infection of the urogenital tract, an infection of the respiratory tract, like, for example rhinitis, tonsillitis, pharyngitis, bronchitis, pneumonia, an infection of the inner organs, like, for example, nephritis, hepatitis, peritonitis, endocarditis, meningitis, osteomyelitis, an infection of the eyes, the ears as well as a cutaneous and a subcutaneous infection, diarrhea, skin disorders, toxic shock syndrome, bacteraemia, sepsis, and tuberculosis.

In yet another preferred embodiment of the invention, in combination with any one of the embodiments listed above or below, the pathogenous bacteria are selected from the genus *Staphylococcus, Streptococcus, Pseudomonas, Escherichia, Salmonella, Helicobacter, Neisseria, Campylobacter, Chlamydia, Clostridium, Vibrio, Triponema, Mycobacterium, Klebsiella, Actinomyces, Bacterioides, Bordetella, Borrelia, Brucella, Corynebacterium, Diplococcus, Enterobacter, Fusobacterium, Leptospira, Listeria, Pasteurella, Proteus, Rickettsia, Shigella, Sphaerophorus, Yersinia,* or combinations thereof.

In a further preferred embodiment of the invention, in combination with any one of the embodiments listed above or below, the bacteria are antibiotic-resistant bacteria. "Antibiotic-resistant" as used herein means that neither a bactericide nor a bacteriostatic effect is achieved by common antibiotics like β-lactam antibiotics, tetracyclines, aminoglycosides, macrolide antibiotics, lincosamids, gyrase inhibitors (chinolones), sulfonamids and trimethoprime, glycopepide antibiotics, polypeptide antibiotics and nitroimidazole derivatives when the antibiotic is applied to the bacteria.

In another preferred embodiment, in combination with any one of the embodiments listed above or below, the antibiotic-resistant bacteria represent methicillin-resistant *Staphylococcus aureus.* The term methicillin-resistant is used herein synonymously to "multiresistant" and "vancomycin-resistant".

In a preferred embodiment of the invention, in combination with any one of the embodiments listed above or below, the composition for use is administrated orally, intranasally or topically.

In another preferred embodiment, in combination with any one of the embodiments listed above or below, the composition for use is in the form of a nasal agent, inhalation mixture, aerosol, mouthwash, mouth spray, nose spray or room spray. More preferably, the composition is in form of a mouth spray, a nose spray or a room spray, in particular in form of a nose spray.

In a more preferred embodiment, the composition is in the form of an aerosol or room spray. Preferably a liquid or solid composition according to the invention is used for this. In addition to the extract, the aerosol or room spray can also contain pharmaceutically harmless substances, carrier media and auxiliary agents. The aerosol or room spay can be used for disinfecting objects and rooms with which bacteria come into contact or could potentially come into contact, particularly means of transport of all types in which people, animals and/or foodstuffs are transported. For example, an airplane can be sprayed with the aerosol according to the invention or with the room spray according to the invention before takeoff, in order to prevent the spread of the viruses and consequently to minimize the risk of infection for people. The aerosol or room spray can also be sprayed in the presence of people, e.g. in waiting rooms, because it does not cause any toxic effects whatsoever in people.

In a particularly preferred embodiment, in combination with any one of the embodiments listed above or below, the composition is applied as a nose spray, particularly for the treatment of bacteria induced diseases of the respiratory tract and sinuses. It is preferable for the composition to be in the form of a liquid extract in this case.

In another preferred embodiment, the composition for use according to the invention, in combination with any one of the embodiments listed above is in the form of a tablet, coated tablet, effervescent tablet, capsule, powder, granulate, sugar-coated tablet, lozenge, pill, ampoule, drop, suppository, emulsions, ointments, gels, tinctures, pastes, creams, moist compresses, gargling solution or plant juice.

As for the oral application, in combination with any one of the embodiments listed above or below, the composition is preferably administered in the form of a tablet. It is preferable for the composition to be in the form of a dry extract in this case.

In another preferred embodiment, in combination with any one of the embodiments listed above or below, the composition can be used according to the invention as a solution, in particular, a gargling solution, mouthwash or tincture, particularly for the treatment of bacterial infections of mouth and upper throat.

Suppositories, in combination with any one of the embodiments listed above or below, represent a preferred embodiment for rectal and vaginal administration of the composition according to the invention.

As for the topical administration, the composition for use according to the invention is administered in the form of emulsions, ointments, gels, tinctures, pastes, creams or moist compresses. In this case, the composition is preferably used in the form of an extract in which the active substances are withdrawn from the plant by means of extraction with a fat, wax or oil. It is furthermore preferred for this extract to be further processed into a dry extract, which is subsequently mixed with or dissolved in a fat, wax or oil.

The concentration of the composition in the application form varies, depending on the type of application. As a rule, the quantity of the composition amounts to between 0.5 and 1,000 mg per dosing unit for solid application forms. Preferably the quantity of the composition amounts to between 1 and 500 mg per unit. In liquid application forms, the composition can be in a concentration of 1 µg/ml to 100 mg/ml, preferably from 25 µg/ml to 50 mg/ml. In the case of semi-solid application forms, the content of the composition amounts to 1 to 90% by weight, preferably 5 to 75 % by weight.

Further elements, such as vitamins and minerals, can be added to the composition used according to the invention.

The composition can, for example, also be added to animal feed or foodstuffs, such as drinks. In the form of an extract, the composition itself can also be infused as tea. It is also possible, however, for hot water to be poured directly over the plant parts, for example, the leaves of the *origanum* plants, for tea preparation. Furthermore, the composition can be a constituent of food supplements, whose ingestion in the winter months can contribute to strengthening the body's defenses and to treat a bacterial infection.

The following examples explain the present invention.

### General procedure for the production of liquid and dry extracts:

The collected plant material is visually checked, and non-original, damaged or eaten away parts are removed.

The purified material is spread on a table in a green house and covered with paper for drying. The plant parts are turned around on a daily basis and visually checked for non-original and damaged parts, which are removed. The residual moisture of the plant material is determined on regular basis. The material is good for further processing when the residual water content is at a maximum of 10%.

The plant material is coarsely cut and transferred to a beaker; Cold, distilled water is added (10 to 30-times the quantity of the plant material). The resulting mixture is heated on a hotplate while being stirred until it starts boiling. Simmering is continued for 1 h.

The hot liquid is strained, and the residual plant material is squeezed out. The resulting aqueous extract is filled directly in a bottle.

For the production of the dry extract, the liquid extract is filled in metal bowls and concentrated at 80°C in a drying oven until the solvent has completely evaporated. The residue is scraped out of the metal bowl and weighed. Alternatively, the liquid extract is freeze-dried according to standard procedures known to the person skilled in the art.

### Preparation of an extract of Origanum

All above-ground parts of *Origanum vulgare* are used for the extraction.

Following the general procedure, 150 g of coarsely cut *Origanum vulgare* is heated in 1500 ml of distilled water for 1 h. The resulting aqueous extract is filled directly in a bottle.

### Preparation of an extract of Ribes nigrum

The leaves of *Ribes nigrum* are used for the extraction.

Following the general procedure, 26.7 g of coarsely cut *Ribes nigrum* leaves are heated in 800 ml of distilled water for 1 h. The resulting aqueous extract is filled directly in a bottle.

### Preparation of an extract of Cistus incanus

Blossoms and little twigs of *Cistus incanus* are used for the extraction.

Following the general procedure, 100 g of coarsely cut *Cistus incanus* material is heated in 1500 ml of distilled water for 1 h. The resulting aqueous extract is filled directly in a bottle.

### Antibacterial effect of Origanum vulgare, Ribes nigrum and Cistus incanus

### Origanum vulgare (Test substance A)

Formulation: 5 mg/ml in H₂O; substance solid (dry frozen)

### Test system:

### Test model: Staphylococcus aureus (USA300 = MRSA) on Mueller-Hinton agar (MH-agar)

### Test groups: Substance A (5 mg)

Procedure: The dry-frozen extract was dissolved in water. 100µl of MRSA solution was plated on MH agar. Subsequently, holes of 8 mm where punched out of the MH agar and filled with 100µl extract solution. After 22 h at 37 °C, the holes were checked optically for inhibition of growth.
Application of MRSA bacteria: 10⁻³/1.63x10⁵/100µl/agar plate
Overnight MRSA culture: 101µl in 8 ml TBS medium, 22 h, 37 °C, shake

### Ribes nigrum (Test substance B)

Formulation: 5 mg/ml in H₂O; substance solid (dry frozen)

### Test system:

Test model: *Staphylococcus aureus* (USA300 = MRSA) on Mueller-Hinton agar (MH-agar)

Test groups: Substance **B** (5 mg)

Procedure: The dry-frozen extract was dissolved in water. 100µl of MRSA solution was plated on MH agar. Subsequently, holes of 8 mm where punched out of the MH agar and filled with 100µl extract solution. After 22 h at 37 °C, the holes were checked optically for inhibition of growth.
Application of MRSA bacteria: 10⁻³/1.63×10⁵/100µl/agar plate
Overnight MRSA culture: 101µl in 8 ml TBS medium, 22 h, 37 °C, shake

### Cistus incanus (Test substance C)

Formulation: 2.5 mg/ml in H₂O; substance solid (dry frozen)

### Test system:

### Test model: Staphylococcus aureus (USA300 = MRSA) on Mueller-Hinton agar (MH-agar)

### Test groups: Substance C (2.5 mg)

Procedure: The dry-frozen extract was dissolved in water. 100µl of MRSA solution was plated on MH agar. Subsequently, holes of 8 mm where punched out of the MH agar and filled with 100µl extract solution. After 22 h at 37°C, the holes were checked optically for inhibition of growth.
Application of MRSA bacteria: 10⁻³/1.63×10⁵/100µl/agar plate
Overnight MRSA culture: 101µl in 8 ml TBS medium, 22 h, 37 °C, shake

### Synergystic antibacterial effect of Origanum vulgare and Ribes nigrum

### Test objects:

Test substances: **A** (= *Origanum vulgare)* + **B** *(= Ribes nigrum)*

Formulation: Substance A: 5 mg/ml in H₂O; substance solid (dry frozen); Substance B: 5 mg/ml in H₂O; substance solid (dry frozen)

### Test system:

Test model: *Staphylococcus aureus* (USA300 = MRSA) on Mueller-Hinton agar (MH-agar)

Test groups: Substance **A** (5 mg) + Substance **B** (5 mg)

Procedure: The dry-frozen substances were dissolved in water. 100µl of MRSA solution was plated on MH agar. Subsequently, holes of 8 mm where punched out of the MH agar and filled with 100µl substance solution. After 22 h at 37°C, the holes were checked optically for inhibition of growth.

Application of MRSA bacteria: 10⁻³/1.63×10⁵/100µl/agar plate

Overnight MRSA culture: 101µl in 8 ml TBS medium, 22 h, 37°C, shake

### Result:

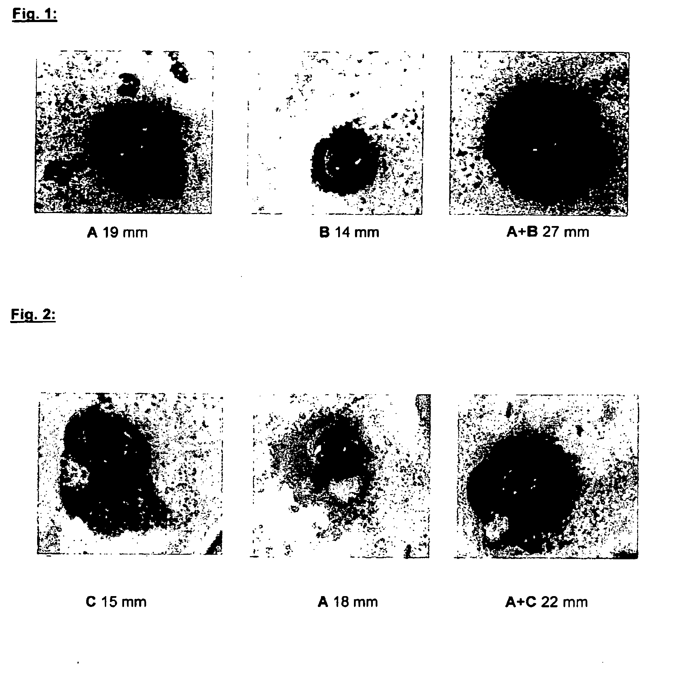

As can be seen from **Fig.** 1, a better inhibition of the growth of MRSA is obtained when using the combination of *Origanum vulgare* and *Ribes nigrum.*

### Synergystic antibacterial effect of Origanum vulgare and Cistus incanus

Test substances: **A** (= *Origanum vulgare)* + **C** (= *Cistus)*

Formulation: Substance **A:** 5 mg/ml in H₂O; substance solid (dry frozen); Substance **C:** 2.5 mg/ml in H₂O; substance solid (dry frozen)

### Test system:

Test model: *Staphylococcus aureus* (USA300 = MRSA) on Mueller-Hinton agar (MH-agar)

Test groups: Substance **A** (5 mg) + Substance C (2.5 mg)

Procedure: The dry-frozen substances were dissolved in water. 100µl of MRSA solution was plated on MH agar. Subsequently, holes of 8 mm where punched out of the MH agar and filled with 100µl substance solution. After 22 h at 37°C, the holes were checked optically for inhibition of growth.
Application of MRSA bacteria: 10⁻³/1.63x10⁵/100µl/agar plate
Overnight MRSA culture: 10µl in 8 ml TBS medium, 22 h, 37°C, shake

### Result:

As can be seen from **Fig. 2****,** a better inhibition of the growth of MRSA is obtained when using the combination of *Origanum vulgare* and *Cistus incanus.*

## Claims

1. Composition for use in treating bacterial infections, comprising a concentrate or an extract of origanum in combination with at least one concentrate or extract of another plant, wherein the plant is selected from plants of the family *Grossulariaceae* and *Cistaceae.*

2. The composition for use according to claim 1, wherein the concentrate or extract is from the aboveground parts of the plant.

3. The composition for use according to claim 1, wherein the plant is selected from the genus *Ribes* and *Cistus.*

4. The composition for use according to claim 3, wherein the plant is selected from *Ribes nigrum, Ribes rubrum* and *Cistus incanus.*

5. The composition for use according to any one of claims 1 to 4, wherein the pathogenous bacteria are selected from the genus *Staphylococcus, Streptococcus, Pseudomonas, Escherichia, Salmonella, Helicobacter, Neisseria, Campylobacter, Chlamydia, Clostridium, Vibrio, Triponema, Mycobacterium, Klebsiella, Actinomyces, Bacterioides, Bordetella, Borrella, Brucella, Corynebacterium, Diplococcus, Enterobacter, Fusobacterium, Leptospira, Listeria, Pasteurella, Proteus, Rickettsia, Shigella, Sphaerophorus, Yersinia,* or combinations thereof.

6. The composition for use according to claim 5, wherein the bacteria are antibiotic-resistant bacteria.

7. The composition for use according to claim 6, wherein the antibiotic-resistant bacteria are Methicillin-resistant *Staphylococcus aureus.*

8. The composition for use according to any one of claims 1 to 7, wherein the composition is in liquid, dry or semi-solid form.

9. The composition for use according to any one of claims 1 to 8, wherein the extract is an aqueous extract or an alcoholic extract.

10. The composition for use according to any one of claims 1 to 9, wherein the composition is administered orally, intranasally or topically.

11. The composition for use according to any one of claims 1 to 10, wherein the composition is in the form of a nasal agent, inhalation mixture, aerosol, mouthwash, mouth spray, nose spray or room spray.

12. The composition for use according to any one of claims 1 to 10, wherein the composition is in the form of a tablet, coated tablet, effervescent tablet, capsule, powder, granulate, sugar-coated tablet, lozenge, pill, ampoule, drop, suppository, emulsions, ointments, gels, tinctures, pastes, creams, moist compresses, gargling solution or plant juice.

13. The composition for use according to any one of claims 1 to 12, wherein the bacterial infection is an infection of the gastrointestinal tract, an infection of the urogenital tract, an infection of the respiratory tract, including rhinitis, tonsillitis, pharyngitis, bronchitis, pneumonia, an infection of the inner organs, including nephritis, hepatitis, peritonitis, endocarditis, meningitis, osteomyelitis, an infection of the eyes, the ears as well as a cutaneous and a subcutaneous infection, diarrhea, skin disorders, toxic shock syndrome, bacteraemia, sepsis, and tuberculosis.

## Patentansprüche

1. Zusammensetzung zur Verwendung in der Behandlung von bakteriellen Infektionen, umfassend ein Konzentrat oder einen Extrakt aus Origanum in Kombination mit mindestens einem Konzentrat oder Extrakt einer anderen Pflanze, wobei die Pflanze ausgewählt ist aus Pflanzen aus der Familie *Grossulariaceae* und *Cistaceae.*

2. Zusammensetzung zur Verwendung nach Anspruch 1, wobei das Konzentrat oder der Extrakt von den oberirdischen Teilen der Pflanze ist.

3. Zusammensetzung zur Verwendung nach Anspruch 1, wobei die Pflanze ausgewählt ist aus der Gattung *Ribes* und *Cistus.*

4. Zusammensetzung zur Verwendung nach Anspruch 3, wobei die Pflanze ausgewählt ist aus *Ribes nigrum, Ribes rubrum* und *Cistus incanus.*

5. Zusammensetzung zur Verwendung nach einem der Ansprüche 1 bis 4, wobei die pathogenen Bakterien ausgewählt sind aus der Gattung *Staphylococcus, Streptococcus, Pseudomonas, Escherichia, Salmonella, Helicobacter, Neisseria, Campylobacter, Chlamydia, Clostridium, Vibrio, Triponema, Mycobacterium, Klebsiella, Actinomyces, Bacterioides, Bordetella, Borrelia, Brucella, Corynebacterium, Diplococcus, Enterobacter, Fusobacterium, Leptospira, Listeria, Pasteurella, Proteus, Rickettsia, Shigella, Sphaerophorus, Yersinia* oder Kombinationen davon.

6. Zusammensetzung zur Verwendung nach Anspruch 5, wobei die Bakterien Antibiotikaresistente Bakterien sind.

7. Zusammensetzung zur Verwendung nach Anspruch 6, wobei die Antibiotika-resistenten Bakterien Methicillin-resistente *Staphylococcus aureus* sind.

8. Zusammensetzung zur Verwendung nach einem der Ansprüche 1 bis 7, wobei die Zusammensetzung in flüssiger, trockener oder halbfester Form vorliegt.

9. Zusammensetzung zur Verwendung nach einem der Ansprüche 1 bis 8, wobei der Extrakt ein wässriger Extrakt oder ein alkoholischer Extrakt ist.

10. Zusammensetzung zur Verwendung nach einem der Ansprüche 1 bis 9, wobei die Zusammensetzung oral, intranasal oder topisch verabreicht wird.

11. Zusammensetzung zur Verwendung nach einem der Ansprüche 1 bis 10, wobei die Zusammensetzung in der Form eines Nasenmittels, Inhalationsgemischs, Aerosols, Mundwassers, Mundsprays, Nasensprays oder Raumsprays vorliegt.

12. Zusammensetzung zur Verwendung nach einem der Ansprüche 1 bis 10, wobei die Zusammensetzung in der Form von einer Tablette, beschichteten Tablette, Brausetablette, Kapsel, Pulver, Granulat, zuckerbeschichteten Tablette, Lutschtablette, Pille, Ampulle, Drops, Zäpfchen, Emulsionen, Salben, Gels, Tinkturen, Pasten, Cremes, feuchte Kompressen, Gurgellösung oder Pflanzensaft vorliegt.

13. Zusammensetzung zur Verwendung nach einem der Ansprüche 1 bis 12, wobei die bakterielle Infektion eine Infektion des gastrointestinalen Trakts, eine Infektion des urogenitalen Trakts, eine Infektion des Respirationstrakts einschließlich Rhinitis, Tonsillitis, Pharyngitis, Bronchitis, Lungenentzündung, eine Infektion der inneren Organe einschließlich Nephritis, Hepatitis, Peritonitis, Endocarditis, Meningitis, Osteomyelitis, eine Infektion der Augen, der Ohren sowie eine kutane und eine subkutane Infektion, Diarrhoe, Hautstörungen, toxisches Schocksyndrom, Bakteriämie, Sepsis und Tuberkulose ist.

## Revendications

1. Composition pour utiliser dans le traitement d'infections bactériennes, comprenant un concentre ou un extrait d'origan combine avec au moins un concentre ou extrait d'une autre plante, dans laquelle la plante est sélectionnée parmi les plantes de la famille *Grossulariaceae* et *Cistaceae.*

2. La composition pour utiliser selon la revendication 1, dans laquelle le concentré ou l'extrait vient des parties hors sol de la plante.

3. La composition pour utiliser selon la revendication 1, dans laquelle la plante est sélectionnée parmi les genres *Ribes* et *Cistus.*

4. La composition pour utiliser selon la revendication 3, dans laquelle la plante est sélectionnée parmi *Ribes nigrum, Ribes rubrum* et *Cistus incanus.*

5. La composition pour utiliser selon une quelconque des revendications 1 à 4, dans laquelle la bactérie pathogène est sélectionnée parmi les genres *Staphylococcus, Streptococcus, Pseudomonas, Escherichia, Salmonella, Helicobacter, Neisseria, Campylobacter, Chlamydia, Clostridium, Vibrio, Triponema, Mycobacterium, Klebsiella, Actinomyces, Bacterioides, Bordetella, Borrelia, Brucella, Corynebacterium, Diplococcus, Enterobacter, Fusobacterium, Leptospira, Listeria, Pasteurella, Proteus, Rickettsia, Shigella, Sphaerophorus, Yersinia,* ou des combinaisons de ces derniers.

6. La composition pour utiliser selon la revendication 5, dans laquelle la bactérie est une bactérie résistante aux antibiotiques.

7. La composition pour utiliser selon la revendication 6, dans laquelle les bactéries résistantes aux antibiotiques sont résistantes à la Méthicillin *Staphylococcus aureus.*

8. La composition pour utiliser selon une quelconque des revendications 1 à 7, dans laquelle la composition est un liquide, ou sous forme solide ou semi-solide.

9. La composition pour utiliser selon une quelconque des revendications 1 à 8, dans laquelle l'extrait est un extrait aqueux ou un extrait alcoolique.

10. La composition pour utiliser selon une quelconque des revendications 1 à 9, dans laquelle la composition est administrée oralement, par voie intra-nasale ou topique.

11. La composition pour utiliser selon une quelconque des revendications 1 à 10, dans laquelle la composition est sous la forme d'un agent nasal, un mélange pour inhalation, un aérosol, un rince-bouche, un spray buccal, un spray nasal, ou un spray parfumé.

12. La composition pour utiliser selon une quelconque des revendications 1 à 10, dans laquelle la composition est sous la forme de comprimés, comprimé enrobé, comprimé effervescent, capsule, poudre, granulé, comprimé à enrobage sucré, pastille, pillule, ampoule, goutte, suppositoire, émulsions, ointements, gels, teintures, pates, crèmes, compresses humides, solution pour se gargariser ou jus de plante.

13. La composition pour utiliser selon une quelconque des revendications 1 à 12, dans laquelle l'infection bactérienne est une infection du tube gastro-intestinal, une infection de des voies urogénitales, une infection des voies respiratoires, incluant la rhinite, l'amygdalite, la pharyngite, la bronchite, la pneumonie, une infection des organes internes, incluant la néphrite, l'hépatite, la péritonite, l'endocardite, la méningite, l'ostéomyélite, une infection des yeux, des oreilles aussi bien qu'une infection cutanée et sub-cutanée, une diarrhée, des troubles de la peau, un syndrome de choc toxique, une bactériémie, une septicémie et une tuberculose.
